# EUROPEAN PATENT SPECIFICATION

(11) **EP 1 951 171 B1**
(45) Date of publication and mention of the grant of the patent: **11.08.2010**
(21) Application number: 06828835.6
(22) Date of filing: 19.10.2006
(51) Int. Cl.: A61F 5/37, A47D 15/00

(54) **HARNESS SYSTEM FOR MATTRESSES**
GURTSYSTEM FÜR MATRATZEN
SYSTEME DE HARNAIS POUR MATELAS

(30) Priority: 20.10.2005 GB 0521364
(43) Date of publication of application: 06.08.2008
(73) Proprietor: Walsh-Barltrop, Sharon, Coagh, County Tyrone BT80 0BR (GB)
(72) Inventor: Walsh-Barltrop, Sharon, Coagh, County Tyrone BT80 0BR (GB)
(74) Representative: Wallace, Alan Hutchinson
(86) International application number: PCT/EP2006/010083
(87) International publication number: WO 2007/045467

(56) References cited:
- WO-A-93/17606
- WO-A-94/13177
- WO-A-98/26695
- WO-A2-2005/110162
- US-A- 3 987 505
- US-A- 4 202 052
- US-A1- 2005 028 278

## Description

### FIELD OF THE INVENTION

The present invention relates to bedding. In particular the present invention relates to a harness system for maintaining a person in a desired position on a mattress.

### BACKGROUND TO THE INVENTION

It is currently recommended that babies and infants sleep on their backs, preferably at the foot of a cot or bed. However, infants in a cot can inadvertently roll onto their front or under the sheets and potentially endanger themselves. Moreover, older children commonly fall from bed and sustain injury. It would be desirable to provide a system that mitigates these problems.

United States Patent application US 2005/0028278 (Landry) discloses a harness system for a mattress having the features recited in the pre-characterising portion of claim 1. However, Landry does not allow the position of an infant to be readily adjusted in a head-to-toe direction.

### SUMMARY OF THE INVENTION

A first aspect of the invention provides a harness system as claimed in claim 1.

A second aspect of the invention provides a combined mattress and harness system as claimed in claim 10.

The preferred arrangement is such that, when fitted to a mattress, the loop extends around a first (typically transverse) axis of the mattress, the harness being arranged so that in use, the person lies substantially perpendicular to the first axis of the mattress, the mattress cover being revolvable relative to the mattress in a direction substantially perpendicular with said first axis to adjust the position of the harness on the mattress.

The loop or mattress cover can be dimensioned to fit different sizes and shapes of mattress, from, for example, a cot mattress to a conventional single or double sized mattress. Additionally the loop or mattress cover may be made from material such as cotton or any other suitable material, typically textiles.

The harness may comprise at least one chest strap and at least one, preferably two, shoulder straps. It is preferable if the at least one chest strap and the at least one, preferably two, shoulder straps can be adjusted separately to suit an individual's size.

The harness may be coupled to the loop or mattress cover at a midpoint, along the width of the loop. The coupling of the harness to the mattress cover may be permanent or releasable to allow for example, washing or replacing of the harness and loop mattress cover separately.

The mattress cover and harness system can include a securing strap for securing the mattress cover to the mattress and/or to a bed frame.

In use the harness system can be rotated relative to the mattress to adjust the position of the harness with respect to the mattress. This allows a person of a particular height to lie on the mattress at a desired location. For example, a person can lie with their feet at the foot of the mattress/bed or alternatively with their head at the head of the mattress/bed.

The harness system may be used to maintain a person, such as an infant, child or disabled person in a desired location and/or orientation on a mattress.

Further advantageous aspects of the invention will become apparent to those ordinarily skilled in the art upon review of the following description of a specific embodiment and with reference to the accompanying drawings.

### BRIEF DESCRIPTION OF THE DRAWINGS

An embodiment of the invention is now described by way of example and with reference to the accompanying drawings in which like numerals are used to indicate like parts and in which:
FIGURE 1 shows a perspective view of a mattress and a mattress cover suitable for use in harness systems embodying the invention.
FIGURE 2 shows a plan view of a harness system embodying the invention.
FIGURE 3 shows a perspective view of the harness system of Figure 2, in use; and
FIGURE 4 shows a side view of the harness system in use.

### DESCRIPTION OF THE INVENTION

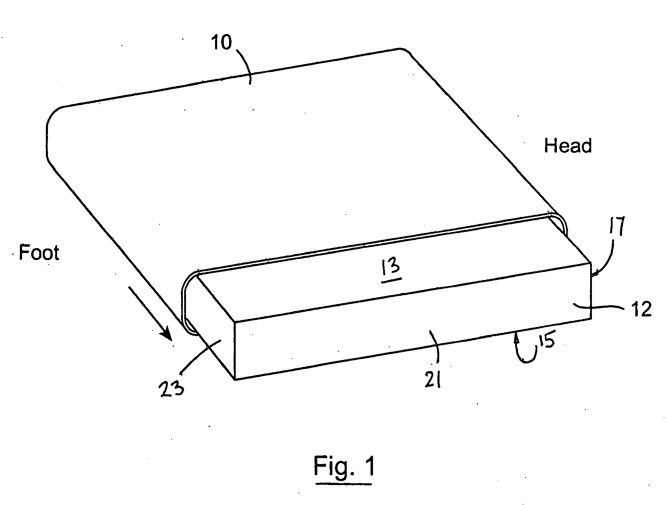

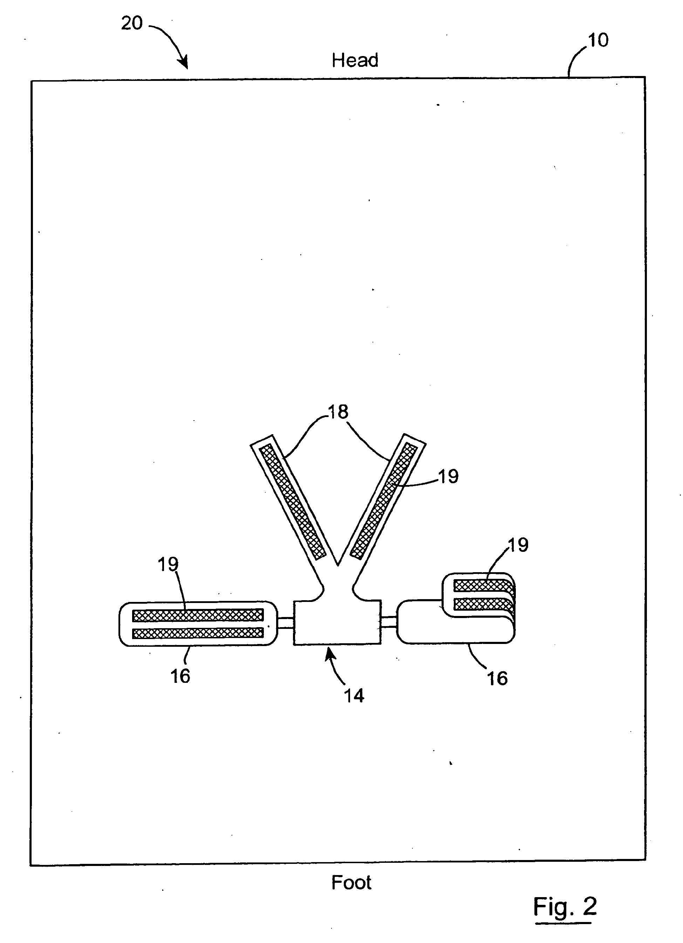

Referring now to Figure 1 of the drawings, there is shown, indicated as 10, a mattress cover in the form of an endless loop or length of material. The loop is typically, but not necessarily, open sided and may conveniently be formed from a sheet of material e.g., a cotton sheet. The mattress cover 10 forms part of a harness system 20 (Figure 2) embodying the present invention, as described in more detail hereinafter.

In use, mattress cover 10 is fitted around a mattress 12. The cover or loop 10 is preferably shaped and dimensioned to fit the mattress 12 such that it is substantially self-retaining on the mattress, i.e. fitting closely enough to prevent it from falling off the mattress 12 while also allowing it to be revolved around the mattress 12 (as is described hereinafter). The cover or loop 10 may be elasticised (e.g. it may be elasticised along its edges and/or may be formed wholly or partially from an elasticised material) to facilitate fitting it to the mattress 12, although this can hamper the revolution of the cover 10. It is preferred that the loop/cover 10 is non-elasticised.

When fitted, the cover 10 typically runs around the mattress 12 to substantially cover it on both its sleeping face 13 and its reverse face 15. Typically, the cover 10 runs around a transverse axis of the mattress 12 and so covers the head and foot portions 17, 23 of the mattress 12. Preferably, the cover 10 is open sided so that there are no side portions or panels to cover the side faces 21 of the mattress 12, as shown in Figure 1.

Retaining means in the preferred form of a harness 14 (Figure 2) is coupled or attached, permanently or releasably, to the cover 10. The harness 14 is configured to hold a person, especially an infant or child, in a fixed position and/or orientation with respect to the cover 10. The harness 14 may for example comprise at least one chest strap 16 and preferably two shoulder straps 18, each strap having, for example, hook and loop type fastenings 19. The harness 14 or retaining means may take any other form suitable for holding a person in a desired position and may for example comprise a combination of one or more straps and/or one or more support blocks.

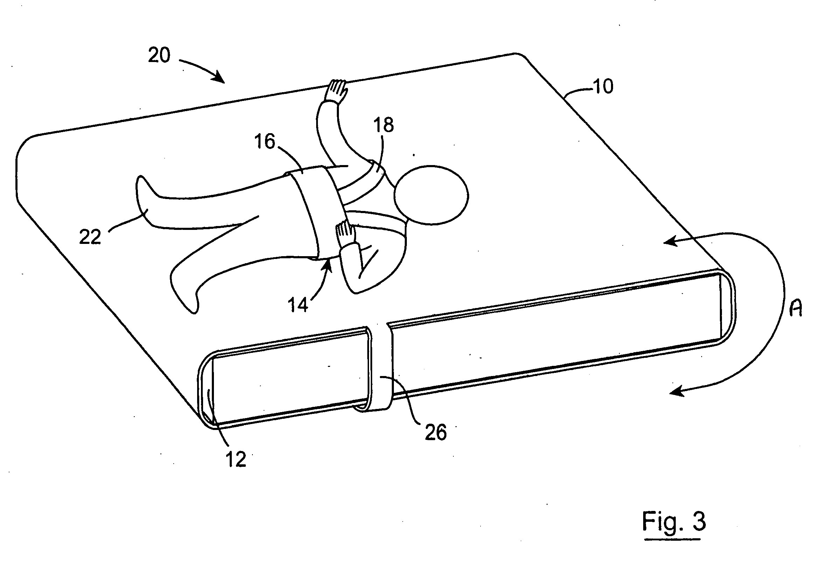

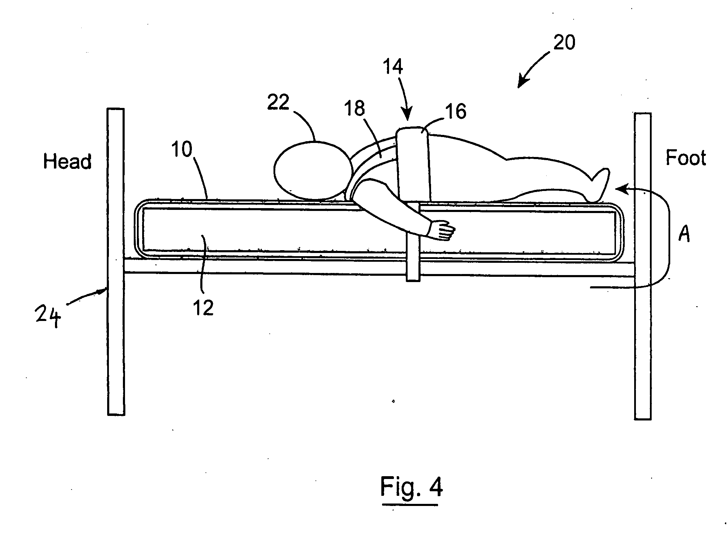

In use, the harness system 20 can be rotated, revolved or otherwise adjusted (as indicated by arrow A) relative to the mattress 12 to adjust the distance between the harness 14 and the head or foot of the mattress 12. This positions the harness 14 at a desired location along the length of the mattress 12. In Figures 3 and 4 the harness 14 is positioned so that an infant, child or other person 22 of a particular height can lie on the mattress 12 with his feet at the foot of the mattress 12/bed 24. Alternatively harness system 20 could be rotated relative to the mattress 12 to position the harness 14 so that the person can lie with their head at the head of the mattress 12/bed 24.

As shown in Figures 3 and 4, the person 22 can be located within the harness 14 and with the chest strap 16 and shoulder straps 18 secured. Hence the system 20 allows the person 22 to be maintained not only at a desired location on the mattress but also in a desired orientation, e.g. lying on their back. The person 22 can remove himself or herself easily, or can readily be removed from, the harness 14 by undoing the straps.

Optionally, a securing strap 26 may be provided for securing the cover 10 to the mattress 12. The strap 26 is particularly intended to prevent further rotation of the cover 10 and so is configured to attach the cover 10 to the mattress 12 by means of, for example, a button or other fastener (not shown). Alternatively, or in addition, one or more further straps or fasteners (not shown) may be provided for preventing the cover 10 from coming off the mattress 12.

In the illustrated embodiment, the loop/cover 10 fits longitudinally around the mattress 12, i.e. around-the normal head and foot of the mattress 12 with the sides of the mattress 12 being exposed. The harness 14 is arranged such that the head-to-toe orientation of the person 22 is substantially parallel with the loop 10 and so, in the present example, the person lies substantially parallel with the longitudinal axis of the mattress 12. In an alternative embodiment, the loop/cover may be shaped and dimensioned to fit transversely around the mattress, i.e. around the mattress from side to side with the normal head and foot of the mattress being exposed by the loop. The harness may be arranged such that the head-to-toe orientation of the person is substantially parallel with the loop which, in this case, means that the person lies substantially parallel with the transverse axis of the mattress 12. The alternative embodiment is particularly useful in cases where more than one person, e.g. twins, is to lie on the mattress since two or more harnesses (or other retaining means) may be located adjacent one another on the cover/loop.

The present invention is not limited to the embodiment(s) described herein, which may be modified or varied without departing from the scope of the present invention.

## Claims

1. A harness system comprising an endless loop of material (10) and means for retaining (14) a person (22) in a substantially fixed position with respect to the endless loop of material, said endless loop of material being shaped and dimensioned to fit around a mattress (12) such that said retaining means (14) is located at a sleeping face (13) of said mattress, **characterised in that** said endless loop of material (10) is open sided and revolvable in use with respect to said mattress (12) to adjust the location of said retaining means (14) with respect to said sleeping face (13) in a first direction, and wherein said retaining means is arranged to hold said person (22) such that their head-to-toe orientation is substantially parallel with said first direction.

2. A harness system as claimed in Claim 1, wherein said endless loop (10) is shaped and dimensioned to substantially cover at least a sleeping face (13) and preferably also a reverse face (15) of said mattress.

3. A harness system as claimed in Claim 2, wherein said endless loop of material comprises a mattress cover.

4. A harness system as claimed in any preceding claim, wherein said retaining means (14) is arranged to hold said person (22) such that their head-to-toe orientation is substantially parallel with said endless loop (10).

5. A harness system as claimed in any preceding claim, wherein said endless loop (10) is formed from one or more textile materials.

6. A harness system as claimed in any preceding claim, wherein said retaining means (14) comprises a harness.

7. A harness system as claimed in any preceding claim, wherein said retaining means comprises at least one block.

8. A harness system as claimed in any preceding claim, wherein said retaining means (14) is fixed to said endless loop of material (10).

9. A harness system as claimed in any one of claims 6 to 8, wherein said harness comprises at least one chest strap (16) and at least one shoulder strap (18) and is arranged to hold said person in a desired orientation lying on their back.

10. A combined mattress and harness system, the harness system comprising an endless loop of material (10) and means for retaining (14) a person (22) in a substantially fixed position with respect to the endless loop of material, the harness system being fitted around said mattress (12) such that said retaining means is located at a sleeping face (13) of said mattress, **characterised in that** said endless loop of material is open sided and revolvable with respect to said mattress to adjust the location of said retaining means with respect to said sleeping face a first direction, and wherein said retaining means is arranged to hold said person such that their head-to-toe orientation is substantially parallel with said first direction.

11. A combined mattress and harness system as claimed in Claim 10, wherein said endless loop of material is shaped and dimensioned to be substantially self-retaining on said mattress.

12. A combined mattress and harness system as claimed in Claim 10, wherein said first direction is substantially parallel with the longitudinal axis of the mattress.

## Patentansprüche

1. Geschirrsystem, das eine endlose Stoffschlaufe (10) und Mittel zum Halten (14) einer Person (22) in einer im Wesentlichen festen Position in Bezug zu der endlosen Stoffschlaufe umfasst, wobei die endlose Stoffschlaufe geformt und bemessen ist, um so um eine Matratze (12) herum zu passen, dass sich das Haltemittel (14) auf einer Schlaffläche (13) der Matratze befindet, **dadurch gekennzeichnet, dass** die endlose Stoffschlaufe (10) seitlich offen und in Gebrauch in Bezug zu der Matratze (12) drehbar ist, um die Position des Haltemittels (14) in Bezug zu der Schlaffläche (13) in einer ersten Richtung anzupassen, und wobei das Haltemittel angeordnet ist, um die Person (22) so zu halten, dass ihre Ausrichtung vom Kopf zu den Füßen im wesentlichen parallel zu der ersten Richtung ist.

2. Geschirrsystem nach Anspruch 1, bei dem die endlose Schlaufe (10) geformt und bemessen ist, um im Wesentlichen mindestens eine Schlaffläche (13) und vorzugsweise auch eine Rückfläche (15) der Matratze zu bedecken.

3. Geschirrsystem nach Anspruch 2, bei dem die endlose Stoffschlaufe eine Matratzenabdeckung aufweist.

4. Geschirrsystem nach einem vorhergehenden Anspruch, bei dem das Haltemittel (14) angeordnet ist, um die Person (22) so zu halten, dass ihre Ausrichtung vom Kopf zu den Füßen im Wesentlichen parallel zu der endlosen Schlaufe (10) ist.

5. Geschirrsystem nach einem vorhergehenden Anspruch, bei dem die endlose Schlaufe (10) aus einem oder mehreren Textilmaterialien gebildet wird.

6. Geschirrsystem nach einem vorhergehenden Anspruch, bei dem das Haltemittel (14) ein Geschirr aufweist.

7. Geschirrsystem nach einem vorhergehenden Anspruch, bei dem das Haltemittel mindestens einen Block aufweist.

8. Geschirrsystem nach einem vorhergehenden Anspruch, bei dem das Haltemittel (14) an der endlosen Stoffschlaufe (10) befestigt ist.

9. Geschirrsystem nach einem der Ansprüche 6 bis 8, bei dem das Geschirr mindestens einen Brustgurt (16) und mindestens einen Schultergurt (18) aufweist und angeordnet ist, um die Person in einer gewünschten Ausrichtung auf dem Rücken liegend zu halten.

10. Kombiniertes Matratzen- und Geschirrsystem, wobei das Geschirrsystem eine endlose Stoffschlaufe (10) und Mittel zum Halten (14) einer Person (22) in einer im Wesentlichen festen Position in Bezug zu der endlosen Stoffschlaufe umfasst, und das Geschirrsystem um die Matratze (12) herum so befestigt ist, dass sich das Haltemittel auf einer Schlaffläche (13) der Matratze befindet, **dadurch gekennzeichnet, dass** die endlose Stoffschlaufe seitlich offen und in Bezug zu der Matratze drehbar ist, um die Position des Haltemittels in Bezug zu der Schlaftläche [in] einer ersten Richtung anzupassen, und wobei das Haltemittel angeordnet ist, um die Person so zu halten, dass ihre Ausrichtung vom Kopf zu den Füßen im Wesentlichen parallel zu der ersten Richtung ist.

11. Kombiniertes Matratzen- und Geschirrsystem nach Anspruch 10, bei dem die endlose Stoffschlaufe geformt und bemessen ist, um im Wesentlichen selbsthaltend auf der Matratze zu sein.

12. Kombiniertes Matratzen- und Geschirrsystem nach Anspruch 10, bei dem die erste Richtung im Wesentlichen parallel zu der Längsachse der Matratze ist.

## Revendications

1. Système de harnais comprenant une boucle sans fin de matière (10) et un moyen de retenir (14) une personne (22) en position sensiblement fixe par rapport à la boucle sans fin de matière, ladite boucle sans fin de matière étant dotée d'une forme et de dimensions aptes à se placer autour d'un matelas (12) de manière à ce que ledit moyen de retenue (14) est situé sur une face de couchage (13) dudit matelas, **caractérisé en ce que** ladite boucle sans fin en matière (10) est ouverte sur ses côtés et peut tourner en cours d'usage par rapport audit matelas (12) pour régler l'emplacement dudit moyen de retenue (14) par rapport à ladite face de couchage (13) dans une première direction, et où ledit moyen de retenue est disposé pour tenir ladite personne (22) de telle manière que son orientation tête-pieds est sensiblement parallèle à ladite première direction.

2. Système de harnais conforme à la revendication 1, où ladite boucle sans fin (10) est dotée d'une forme et de dimensions aptes à couvrir sensiblement au moins une face de couchage (13) et de préférence aussi une face de dos (15) dudit matelas.

3. Système de harnais conforme à la revendication 2, où ladite boucle sans fin de matière comprend une housse de matelas.

4. Système de harnais conforme à une quelconque des revendications précédentes, où ledit moyen de retenue (14) est disposé pour tenir ladite personne (22) de telle manière que son orientation tête-pieds est sensiblement parallèle à ladite boucle sans fin (10).

5. Système de harnais conforme à une quelconque des revendications précédentes, où ladite boucle sans fin (10) est formée d'une ou plusieurs matières textiles.

6. Système de harnais conforme à une quelconque des revendications précédentes, où ledit moyen de retenue (14) comprend un harnais.

7. Système de harnais conforme à une quelconque des revendications précédentes, où ledit moyen de retenue (14) comprend au moins une cale.

8. Système de harnais conforme à une quelconque des revendications précédentes, où ledit moyen de retenue (14) est fixé à ladite boucle sans fin de matière (10).

9. Système de harnais conforme à une quelconque des revendications 6 à 8, où ledit harnais comprend au moins une bretelle thoracique (16) et au moins une bretelle d'épaule (18) et est disposé pour tenir ladite personne couchée sur le dos dans une orientation désirée.

10. Système combiné de matelas et harnais, le système de harnais comprenant une boucle sans fin de matière (10) et un moyen de retenir (14) une personne (22) en position sensiblement fixe par rapport à ladite boucle sans fin de matière, le système de harnais étant installé autour dudit matelas (12) de manière à ce que ledit moyen de retenue est situé sur une face de couchage (13) dudit matelas, **caractérisé en ce que** ladite boucle sans fin de matière est ouverte sur ses côtés et peut tourner par rapport audit matelas pour régler l'emplacement dudit moyen de retenue par rapport à ladite face de couchage [dans] une première direction, et où ledit moyen de retenue est disposé pour tenir ladite personne de telle manière que son orientation tête-pieds est sensiblement parallèle à ladite première direction.

11. Système combiné de matelas et harnais conforme à la revendication 10, où ladite boucle sans fin de matière est dotée d'une forme et de dimensions de manière à rester sensiblement tenue d'elle-même sur ledit matelas.

12. Système combiné de matelas et harnais conforme à la revendication 10, où ladite première direction est sensiblement parallèle à l'axe longitudinal du matelas.
